# EUROPEAN PATENT SPECIFICATION

(11) **EP 2 068 901 B1**
(45) Date of publication and mention of the grant of the patent: **21.11.2018**
(21) Application number: 07818713.5
(22) Date of filing: 05.10.2007
(51) Int. Cl.: A61K 36/63, A61P 21/00

(54) **USE OF HYDROXYTYROSOL TO REDUCE THE AMOUNT OF LACTIC ACID IN PLASMA**
VERWENDUNG VON HYDROXYTYROSOL ZUR REDUKTION DER MENGE VON MILCHSÄURE IN PLASMA
UTILISATION DE HYDROXYTYROSOL POUR DIMINUER LA CONCENTRATION DE LACTATE DANS LE PLASMA

(30) Priority: 05.10.2006 EP 06121809; 05.10.2006 EP 06121812
(43) Date of publication of application: 17.06.2009
(73) Proprietor: DSM IP Assets B.V., 6411 TE Heerlen (NL)
(72) Inventor: RIETJENS, Saskia Johannes, 6221 GJ Maastricht (NL); BAST, Aalt, 3862 XG Nijkerk (NL); HAENEN, Guido Rembertus Michiel Marie, 6245 EV Eijsden (NL); HEYDEN, VAN DER Lucas Cyril Gerard, 2498 BW Den Haag (NL)
(74) Representative: Dux, Roland
(86) International application number: PCT/EP2007/008636
(87) International publication number: WO 2008/040550

(56) References cited:
- EP-A- 1 639 902
- WO-A-03/020026
- WO-A-03/082259
- WO-A1-2006/053872
- JP-A- 58 146 241
- JP-A- 2002 153 238
- JP-A- 2005 058 115
- US-B1- 6 399 116

## Description

This invention relates to the use of olive extracts containing hydroxytyrosol to decrease in healthy humans the amount of lactic acid accumulated in blood plasma, body and muscle cells during exercise and to increase in healthy humans glutathione levels in muscles associated with exercise.

### BACKGROUND OF THE INVENTION

Hydroxytyrosol (HT) has been described for use in a composition to retain or restore muscle health resulting from damage incurred during exercise. See WO 2006/053872 (published May 26, 2006). However, the source of the HT was not specified, and the actions described are generally attributed to the antioxidant ability of the HT. In contrast, it has been found that at least some of the beneficial properties of HT are not attributable to its antioxidant ability.

In the formulation of nutritional products for both human and animal consumption, it is often desirable to use natural ingredients. It has been found, that an all natural olive extract can be used as a substitute source of HT in the manufacture of nutritional products which can promote muscle health, and protect the muscle from exercise-induced damage through mechanisms which are not directly associated with antioxidant activity.

JP 2005 058115 discloses an olive extract to be used for flavoring food and beverage products. JP 58 146241 discloses a chewing gum comprising an olive oil extract. WO 2006/053872 discloses the use of hydroxytyrosol as antioxidant to prevent muscle damage due to exercising. WO 03/020026 discloses the use of a composition comprising extract of artemisia optionally in combination with olive leaf extract to increase muscle cell strength and muscle cell. WO 03/082259 discloses the use of phenolic extracts of olives for the treatment of neurodegenerative diseases such as Alzheimer's Disease. JP 2002 153238 discloses the use of a glycoside which can be extracted from olive in healthy ageing and prevention of arteriosclerosis. US 6,399,116 discloses an extract of *Rhodiola crenulata* that contains a variety of active ingredients which is said to lower lactic acid levels. EP 1 639 902 discloses a proanthocyanidin, particularly from pine bark extract, which can reduce the level of lactic acid upon physical exercise.

### BRIEF DESCRIPTION OF THE INVENTION

The invention is defined by the claims.
This invention relates to the use of olive extracts according to claim 1 and 5. Olive extracts can be used to make nutraceuticals which promote muscle health, and in particular, to protect muscles during exercise, to promote recovery from injuries during exercise, and to relieve muscle soreness connected with exercise. While the olive extract is used as a human food, it can also be applied for animals, particularly those engaged in strenuous exercise or work, such as racing animals (dogs, camels, horses), and animals which pull heavy loads (farm horses, sled dogs and the like).

It has been found, that olive extract can protect muscles in at least two ways which are not directly associated with its anti-oxidant properties
First, olive extract decreases the amount of lactic acid which can accumulate in blood plasma, body and muscle cells during exercise. This allows the participant to exercise or train for a longer period of time, and to exercise more strenuously while minimizing post-exercise soreness.

Secondly, olive extract increases glutathione levels in muscle associated with exercise. This means that the olive extract is activating the body's own anti-oxidant mechanisms, in addition to acting as an anti-oxidant on its own. Thus an olive extract can be used to enhance the body's own antioxidant capability in maintaining muscle health.

Olive extract can be used to promote muscle health by administering an olive extract to an animal (including humans) prior, during exercise, or shortly thereafter in order to maintain muscle health, and to prevent muscle damage incurred during exercise. A nutraceutical composition comprising olive extract is effective in promoting muscle health in an animal, including humans, which is subject to post-exercise muscle soreness, muscle pain, and muscle injury.

### DESCRIPTION OF THE FIGURES

Figure 1 is the time schedule of the study of Example 1.
Figure 2 shows plasma lactate concentration (in % change from baseline) as function of time, * P<0.05 (hydroxytyrosol vs. placebo) for the group of 8 subjects.
Figure 3 compares the concentration of GSH (reduced glutathione), GSSG (oxidized glutathione (dimer)) and GR (glutathione reductase) activity in the two groups, (post-exercise values compared to pre-exercise values) in the 8 subject group.
Figure 4 shows the increase in GSH, GSSH and GR after exercise compared to baseline (means ± SEM for the 8 subject group).

### OLIVE EXTRACT DECREASES LACTIC ACID ACCUMULATION

It has been found that olive extracts decrease the accumulation of lactic acid/lactate concentrations during and upon exercise in plasma, body and muscle. The energy consumption of skeletal muscle cells may increase up to 100-fold when going from rest to high-intensity exercise. This high energy demand can exceed the aerobic capacity of the muscle cells, and a large fraction of the ATP required will have to come from anaerobic metabolism. High-intensity exercise also leads to a rapid decline in contractile function known as skeletal muscle fatigue. Thus, one consequences of anaerobic metabolism is the decline in contractile function.

Anaerobic breakdown of glycogen leads to an intracellular accumulation of inorganic acids, of which lactic acid is quantitatively the most important. Lactic acid, being a strong acid, will dissociate into lactate and hydrogen ions; the lactate itself becomes a source of energy for muscle cells. Therefore acidification of muscles lowers muscular power production and produces fatigue and muscle pain. Increased lactate production coincides with cellular acidosis and remains a good indirect marker for cell metabolic conditions that induce metabolic acidosis.

As lactate and hydrogen ions build up in cells during longer exercise or physical work, a 'co-transporter' system removes hydrogen ions out of muscle cells, thus preserving relatively favorable pH conditions by preventing hydrogen ions from accumulating in the muscle. Endurance training improves the capacity of this co-transport system.

The hydrogen transportation system is not the only way that muscle cells can prevent acid build-up during exercise. There is also the Na+/H+ exchange system, which basically pumps hydrogen ions out of muscle cells and brings sodium ions in to replace them (lactate does not participate in this process). Like the hydrogen transportation system, this exchange consumes energy, and the Na+/H+ exchange appears to be critically important during exercise.

During intense muscle activity, the intracellular pH may fall by approximately 0.5 pH units. There are two major lines of evidence that have been used to link this decline in pH to the contractile dysfunction in fatigue. First, studies on human muscle fatigue have often shown a good temporal correlation between the decline of muscle pH and the reduction of force or power production. Second, studies on skinned skeletal muscle fibers have shown that acidification may reduce both the isometric force and the shortening velocity (Hakan Westerblad et al 2002 News Physiol Sci 17: 17-21).

Up to now relatively little is known, except for exercise, how the hydrogen ion presence in muscle can be positively influenced. Although there is little known on the mechanism, it is found that the intracellular pH of skeletal muscle decreases as lactic acid accumulates. A decrease of pH or the presence of lactic acid can be felt as muscle fatigue. Possible explanations for this phenomenon can be that low pH or the presence of lactic acid inhibits enzymes such as phosphofructokinase which plays a role in the maintenance of the muscle energy supply. Some articles make a link between low pH or lacic acid presence and reduced muscle performance such as output force.

Surprisingly it has been found, in accordance with this invention, that the intake of hydroxytyrosol in the form of an olive extract, decreases the lactic acid content in blood plasma, body and muscle cells.

The olive extract reduces muscle fatigue and muscle pain, and post-exercise muscle soreness which are related with high lactate content in the muscle. Thus olive extracts containing hydroxytyrosol can be used to prevent, or decrease muscle fatigue, to prevent or reduce muscle pain associated with exercise, and to allow faster recovery from muscle fatigue, muscle pain, or post-exercise muscle soreness.

Post-exercise muscle fatigue, muscle pain, and muscle soreness due to lactic acid build up can be present after physical exercise. By preventing the effect of pH decrease in muscle cells, the use of olive extracts containing hydroxytyrosol results in an improved muscle health by the lowering of the muscle fatigue and therefore enables an improved muscle performance. The latter effect is especially noticeable on long-term, physical or sports exercise, so preferably in circumstances wherein the exercise takes between 0.5 to 8 hours, preferably between 0.5 and 2 hours time.

We noted that preferably the olive extract containing hydroxytyrosol is taken before or during exercise. Preferably hydroxytyrosol is orally consumed, but is not a chewing gum.

In one trial, hydroxytyrosol was consumed 10 minutes to 1 hour before the exercise, and the lactate level in blood plasma was seen to decrease during and after the exercise compared to a reference experiment wherein a placebo was used.

Hydroxytyrosol containing olive extract is used to decrease and prevent muscle cramping, and to allow a faster recovery from muscle cramping. This cramping may result from physical stress (for example exercise), mental stress (for example stress at work or examinations) or from stress related diseases like Repetitive Strain Injury (RSI).

RSI refers to a wide range of musculoskeletal injuries such as carpal tunnel syndrome, bursitis or tendonitis. It also covers Work-related Upper Limb Disorders, Occupational Overuse Injuries, or Cumulative Trauma Disorders. These injuries can occur, for example, in employees engaged in heavy computer keyboard use. Another term sometimes used for these types of injuries is repetitive motion disorder (RMI), an overuse syndrome associated with loss of function in a limb resulting from repetitive movement or sustained static loading.

Advantageously olive extracts containing hydroxytyrosol can be used for the manufacture of a nutraceutical, preferably a medicament for the decrease of the lactate level in blood plasma, muscle or body and/or to prevent or decrease muscle fatigue, muscle pain, muscle soreness, or muscle cramps, or to recover faster from post-exercise muscle fatigue, muscle pain, muscle soreness or muscle cramps. The olive extracts of this invention are helpful in case of a performance of an elite athlete as well as after an exercise or performance of a less-well trained person.

### GLUTATHIONE LEVELS

The present invention also relates to use of hydroxytyrosol containing olive extracts to increase the glutathione level present in blood plasma, body and muscle during and after exercise.

Glutathione is a tripeptide amino acid produced in the liver primarily from cysteine. It acts as a cellular antioxidant by inhibiting free radical proliferation. Antioxidants work in a variety of ways to reduce the effects of free radicals in muscle cells. They may work by decreasing the damage caused by free radicals, stopping them from forming to begin with, or by oxidizing them by combining with them and neutralizing their harmful effects through stabilization. The intake of antioxidants can directly affect free radicals in the muscle. Glutathione is a cellular antioxidant that has been shown to react on the free radicals in the muscle and which is already present in the body. It is mostly present in its reduced form, glutathione (GSH). The glutathione cycle eliminates H2O2 (hydrogenperoxide) in a reaction catalyzed by GSH peroxidase:

2 GSH + H2O2 → GSSG (oxidized form)+ 2 H2O

The absence or reduced function of this defense system makes the muscle cell vulnerable to oxidative stress.

"Regular exercise" i.e. that which is not as intense as what is seen in elite or professional athletes may be associated with a compensatory increase in cellular defenses against free radical damage. These defenses involve several mechanisms such as enhanced antioxidant enzyme activity and changes in protective immune responses. These antioxidant enzymes are synthesized in the body and include certain thiols, glutathione and ubiquinone. Important antioxidants, which cannot be synthesized in the body must come from the diet. These include vitamins C, E, and beta carotene. Cross sectional studies indicate that athletes have higher antioxidant enzyme levels than sedentary individuals. If this is to be true, one could conclude that physically active individuals may indeed be more resistant to free radical damage. Training studies have also confirmed that there appears to be a relationship between weekly training distance and antioxidant capacity. The more trained the individuals, the more likely they are able to counteract an increase in free radicals generated by exercise. The "weekend warrior" who may exercise strenuously only on occasion, may be most at risk for oxidative damage to cells. The key message for a nutritionist to understand is that since strenuous exercise may deplete the pool of antioxidant vitamins, nutrient density and quality of the diet must be addressed in order to provide adequate amounts of these vitamins. One cannot assume that a normal balanced diet is always going to be adequate to provide sufficient antioxidant levels.

It has been found that the intake of olive extracts containing hydroxytyrosol influences the glutathione system in blood plasma, body and muscle tissue. Even more surprisingly the olive extracts containing hydroxytyrosol up-regulates the glutathione system in blood plasma, body and muscle tissue during and after exercise, resulting in an increase of the glutathione level. The increase in glutathione level leads to an increase of the total antioxidant level in blood plasma and muscle. As a possible consequence thereof the amount of peroxides present in the muscle tissue will be reduced, which will lead to increased muscle performance.

Olive extracts containing hydroxytyrosol can be used for the manufacture of a nutraceutical, preferably a medicament for increase of the glutathione level in blood plasma, muscle or body and/or muscle performance compared to the level without the use of hydroxytyrosol.

### Formulations

The olive extracts containing hydroxytyrosol can be used in any suitable form such as a food or a beverage, as Food for Special Nutritional Uses, as a dietary supplement, as a nutraceutical or even in feed or pet food.

The olive extract containing hydroxytyrosol may be added at any stage during the normal process of these products. Suitable food products include e.g. cereal bars, bakery items such as cakes and cookies and also liquid foods such as soups or soup powders. Suitable beverages encompass non-alcoholic and alcoholic drinks as well as liquid preparations to be added to drinking water and liquid food. Non-alcoholic drinks are preferably mineral water, sport drinks, near water drinks, fruit juices, lemonades, teas and concentrated drinks such as shots. The sports drinks can be hypotonic, hypertonic or isotonic. Sports drinks can be available in liquid form, as concentrates or as powder (to be dissolved in a liquid, as for example water). Examples of Foods for Special Nutritional Uses include the categories of sport food. slimming foods, infant formula and clinical foods.

The term "dietary supplement" as used herein denotes a product taken by mouth that contains a compound or mixture of compounds intended to supplement the diet. The compound or mixture of compounds in these products may include: vitamins, minerals, herbs or other botanicals and amino acids. Dietary supplements can also be extracts or concentrates, and may be found in many forms such as tablets, capsules, softgels, gelcaps, liquids, or powders.

The term nutraceutical as used herein denotes the usefulness in both the nutritional and pharmaceutical field of application. The nutraceutical may be in any form that is suitable for administrating to the animal body including the human body, especially in any form that is conventional for oral administration, e.g. in solid form such as (additives/supplements for) food or feed, food or feed premix, tablets, pills, granules, dragées, capsules, and effervescent formulations such as powders and tablets, or in liquid form such as solutions, emulsions or suspensions as e.g. beverages, pastes and oily suspensions. Furthermore, a multi-vitamin and mineral supplement may be added to the nutraceutical compositions to obtain an adequate amount of an essential nutrient, which is missing in some diets. The multi-vitamin and mineral supplement may also be useful for disease prevention and protection against nutritional losses and deficiencies due to lifestyle patterns. The nutraceutical can further comprise usual additives, for example sweeteners, flavors, sugar, fat, emulgators, preservatives. The nutrition can also comprise other active components, such as (hydrolysed) proteins as described in for example WO02/45524. Also other antioxidants can be present in the nutrition, for example flavonoids, carotenoids, ubiquinones, rutin, lipoic acid, catalase, glutatione (GSH) and vitamins, such as for example C and E or their precursors.

Hydroxytyrosol is advantageously present in the olive extract in an effective amount. Generally between 1 mg to about 500 mg of hydroxytyrosol in an olive extract is effective per serving to assort an effect. Preferably between 1 mg and 250 mg hydroxytyrosol is present in the olive extract, and even more preferably between1 mg and 100 mg in an olive extract is used

The nutraceutical comprising olive extract containing hydroxytyrosol can be consumed before, during or after the exercise. In the case where it is used before exercise, it is preferably consumed about 20 minutes before. In case where it is used after exercise, it is preferably consumed within one hour thereafter, more preferably immediately after the exercise.

In addition to nutraceutical products suitable for consumption of humans, it is also possible to use hydroxytyrosol in feed for animals including pet food. It is then especially suitable for animals, which are employed for their muscular force, for example (race) horses or dogs (i.e racing dogs or sled dogs).

The following non-limiting examples are presented to better illustrate the invention.

### EXAMPLE 1

### Subjects

Eight healthy male volunteers with no history of participating in any regular exercise program were recruited for the present study. The subjects' characteristics are shown in Table 1, below. All subjects were informed in advance on the nature and possible risks of the experimental procedures before their written informed consent was obtained. This study was approved by the Medical Ethical Review Board of the Academic Hospital Maastricht, the Netherlands.

### Standardization of diet and activity prior to testing

Subjects were instructed not to consume olives, olive oil, olive products, fruit juices, vegetable juices, wine, more than two cups of tea, more than one piece of fruit, more than two serving spoons of vegetables, chocolate and supplements containing antioxidants during three days prior to testing and on the test day itself. The volunteers consumed a standard diet (low in antioxidants) the evening before the test day. Subjects were asked to record their food intake during the entire testing period. All subjects were instructed to refrain from any sort of heavy physical exercise during the entire period except for the resistance exercise session.

### Studies

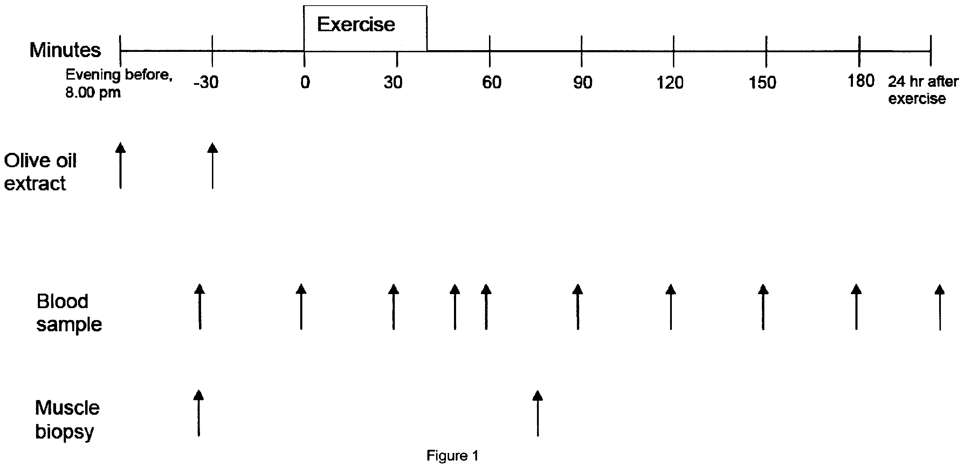

All subjects were studied on two different occasions. Via a randomized cross-over design, subjects received either an olive extract or placebo. Beverages were taken twice, the evening prior to testing (8.00 pm) and 30 minutes before the start of the exercise (Figure 1). The olive extract (DSM Food Specialties, Delft, the Netherlands) contained 200 mg hydroxytyrosol. The placebo was similar in taste and color.

### Resistance exercise

In the present study the same exercise protocol was used as described by Koopman et al. 2005 Eur J Appl Physiol 94:180-187. Subjects arrived at the laboratory at 8.00 am, in an overnight fasted state. Subjects performed a general warm-up of 5 min using a Stairmaster (Jimsa Benelux BV, Rotterdam, the Netherlands). Thereafter, the resistance exercise session targeted the legs, with 8 sets of 10 repetitions on the horizontal leg press machine (Technogym BV, Rotterdam, the Netherlands) and 8 sets of 10 repetitions on the leg extension machine (Technogym). Both exercises were performed at 75% of the subjects' individual 1RM with 2 min rest intervals between sets and in total required approximately 40 minutes to complete. All subjects were verbally encouraged during the test to complete the entire protocol. Energy expenditure during the exercise session was not measured. Based on indirect calorimetry measurements during similar resistance exercise protocols, others have shown energy expenditure rates ranging between 14 and 27 kJ·min⁻¹ (Ballor et al. Am J Clin Nutr (1988) 47: 19-25 and Burleson et al. Med Sci Sports Exerc (1998) 30: 518-522).

### Blood sampling

Blood was collected before the start of the exercise, during exercise and up to two and a half hours after exercise (Figure 1). Blood samples were collected in tubes containing heparin and placed on ice. After centrifugation at 1000 g and 4°C for 5 min, aliquots of plasma were stored at -80°C until analysis.

Blood analyses were directed at markers of damage and inflammation and the level of antioxidants. Markers of damage were malondialdehyde and protein carbonyls and markers of inflammation were NF-κβ, IL-6, IL-10 and TNFα (after stimulation of the blood with LPS). Several antioxidant levels were measured: Trolox Equivalent Antioxidant Capacity (TEAC), uric acid, vitamin E, vitamin C, glutathione (GSH and GSSG), superoxide dismutase, glutathione peroxidase and hydroxytyrosol. Additionally, lactate levels were determined in plasma.

### Muscle biopsies

Muscle biopsy samples were taken 30 minutes before the start of the exercise and 30 minutes after exercise. The second muscle biopsy was taken from the contralateral leg. Muscle biopsies were obtained from the middle region of the m. vastus lateralis (15 cm above the patella) and approximately 3 cm below entry through the fascia using the percutaneous needle biopsy technique (Bergstrom1975 Scand J Clin Lab Invest 35: 518-522). Muscle samples were dissected carefully, freed from any visible non-muscle material and rapidly frozen in liquid nitrogen. A small part of the muscle biopsy was embedded in Tissue-Tek (Sakura Finetek, Zoeterwoude, the Netherlands) and rapidly frozen in liquid nitrogen cooled isopentane. Muscle biopsies were stored at -80°C until analysis.

The following parameters were measured in the muscle biopsies: glutathione (GSH and GSSG), uric acid, superoxide dismutase, glutathione reductase and glutathione S-transferase (Gosker et al. 2005 Respir Med 99(1):118-125).

### Urine collection

Morning urine was collected on the test day and the day after the test day. Aliqouts of urine were frozen at -80°C until analysis of 8-isoprostanes and creatinine.

### Statistics

All data are expressed as means ± SEM. Paired t-tests were used to compare the differences between prior supplementation with the olive extract and placebo. Statistical significance was set at P<0.05.

**Table 1. Subjects' characteristics**

| | Mean ± SEM |
|---|---|
| Age (yrs) | 22.0 ± 1.9 |
| Weight (kg) | 75.4 ± 3.3 |
| Height (m) | 1.82 ± 0.02 |
| BMI (kg·m⁻²) | 22.7 ± 0.8 |
| 1RM leg press (kg) | 197 ± 10 |
| 1RM leg extension (kg) | 117 ± 6 |

### Example 2

### Lactate accumulation.

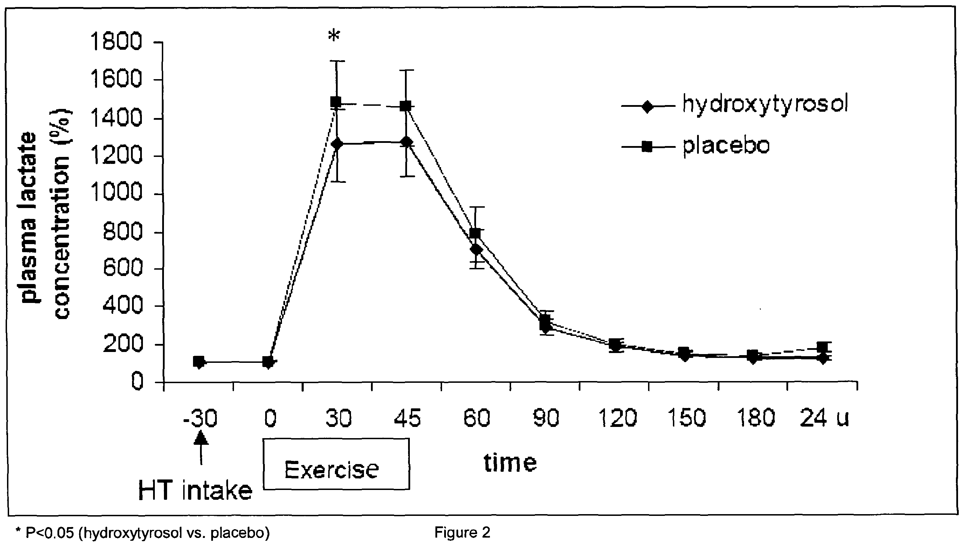

Lactate is produced by anaerobic energy production in muscle. Lactate accumulates in muscle and blood during intensive exercise, which diminishes exercise performance. We showed in our study that the lactate concentration in plasma considerably increases during exercise (Figure 2). The lactate concentration in plasma rose 14.8 fold during exercise, as measured at 30 and 60 minutes after exercise start, respectively. After intake of hydroxytyrosol, the lactate concentration in plasma increased only 12.8 fold during exercise (Figure 2). Hydroxytyrosol decreased the plasma peak concentration of lactate during exercise (Figure 2).

### Example 3

### Glutathione system in muscle

Glutathione is an important element in the antioxidant network. Glutathione is mostly present in its reduced form (GSH). The enzyme glutathione reductase is capable of converting oxidized glutathione (GSSG) into GSH.

Hydroxytyrosol enhances the glutathione antioxidant system in muscle, i.e. the hydroxytyrosol helps to maintain optimal muscle function and to protect muscle from damage from exhaustive exercise by increasing GSH levels, thereby leading to a higher GSH/GSSG ratio.

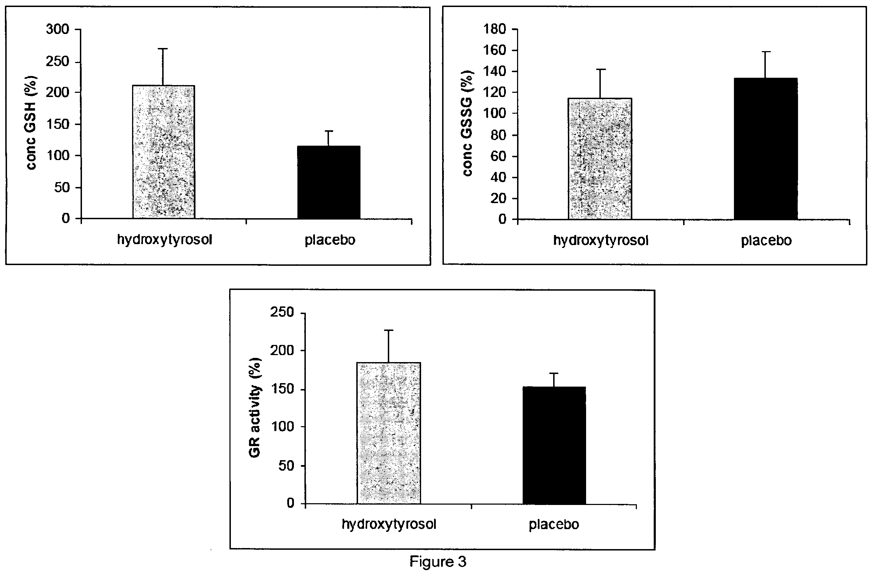

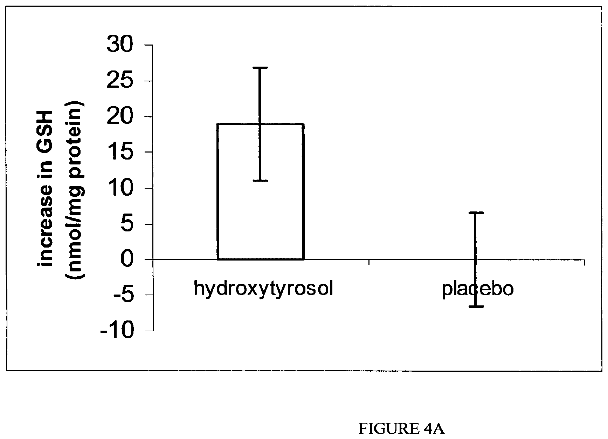

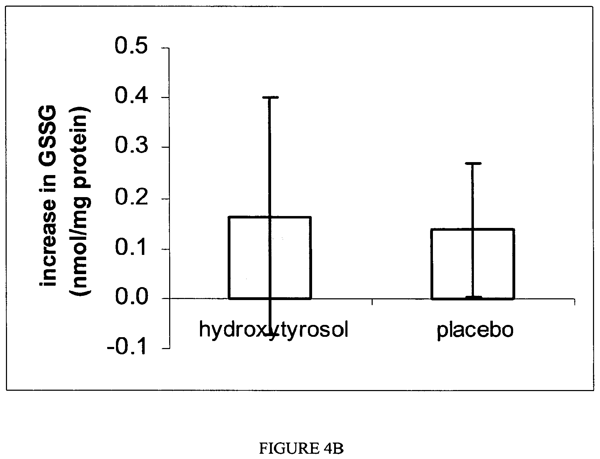

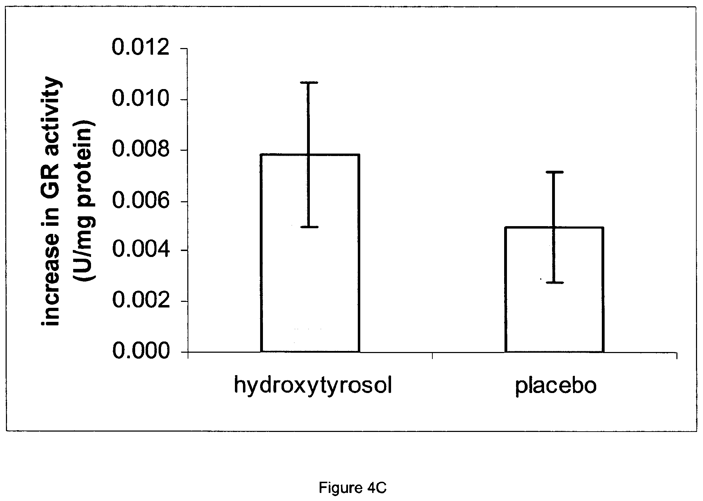

The GSH concentration in muscle after exercise was increased in subjects who were administered hydroxytyrosol, compared to those who were administered placebo (Figures 3 and 4). The concentration of GSSG in muscle was increased less in the hydroxytyrosol group, compared with placebo (Figure 3). Hydroxytyrosol increased the activity of the enzyme glutathione reductase after exercise (Figures 3 and 4). The activity of glutathione reductase is less increased after placebo intake.

## Claims

1. Use of an olive extract containing hydroxytyrosol to decrease in healthy humans the amount of lactic acid accumulated in blood plasma, body and muscle cells during exercise.

2. The use according to claim 1, wherein the amount of hydroxytyrosol in the olive extract is between 1 mg and 250 mg, and preferably between 1 mg and 100 mg.

3. The use according to claim 1 or 2, **characterized in that** olive extract is comprised in a neutraceutical composition.

4. The use according to according to claim 3, **characterized in that** the olive extract is consumed about 20 minutes before exercise.

5. Use of an olive extract containing hydroxytyrosol to increase in healthy humans glutathione levels in muscles associated with exercise.

6. The use according to claim 5, wherein the amount of hydroxytyrosol in the olive extract is between 1 mg and 250 mg, and preferably between 1 mg and 100 mg.

7. The use according to claim 5 or 6, **characterized in that** olive extract is comprised in a neutraceutical composition.

8. The use according to according to claims 7, **characterized in that** the olive extract is consumed about 20 minutes before exercise.

## Patentansprüche

1. Verwendung eines Hydroxytyrosol enthaltenden Olivenextrakts zur Verminderung der Menge an im Blutplasma, im Körper und in Muskelzellen während körperlicher Betätigung angereicherten Milchsäure bei gesunden Menschen.

2. Verwendung nach Anspruch 1, wobei die Menge an Hydroxytyrosol im Olivenextrakt zwischen 1 mg und 250 mg und vorzugsweise zwischen 1 mg und 100 mg liegt.

3. Verwendung nach Anspruch 1 oder 2, **dadurch gekennzeichnet, dass** der Olivenextrakt Bestandteil eines Nutrazeutikums ist.

4. Verwendung nach Anspruch 3, **dadurch gekennzeichnet, dass** der Olivenextrakt etwa 20 Minuten vor der körperlichen Betätigung eingenommen wird.

5. Verwendung eines Hydroxytyrosol enthaltenden Olivenextrakts zum Erhöhen der mit körperlicher Betätigung assoziierten Glutathionspiegel in Muskeln bei gesunden Menschen.

6. Verwendung nach Anspruch 5, wobei die Menge an Hydroxytyrosol im Olivenextrakt zwischen 1 mg und 250 mg und vorzugsweise zwischen 1 mg und 100 mg liegt.

7. Verwendung nach Anspruch 5 oder 6, **dadurch gekennzeichnet, dass** der Olivenextrakt Bestandteil eines Nutrazeutikums ist.

8. Verwendung nach Anspruch 7, **dadurch gekennzeichnet, dass** der Olivenextrakt etwa 20 Minuten vor der körperlichen Betätigung eingenommen wird.

## Revendications

1. Utilisation d'un extrait d'olive contenant de l'hydroxytyrosol, afin de réduire, chez l'homme en bonne santé, la quantité d'acide lactique accumulée dans le plasma sanguin, les cellules du corps et des muscles lors de l'exercice.

2. Utilisation selon la revendication 1, dans laquelle la quantité d'hydroxytyrosol dans l'extrait d'olive se trouve entre 1 mg et 250 mg, et préférablement entre 1 mg et 100 mg.

3. Utilisation selon la revendication 1 ou 2, **caractérisée en ce que** l'extrait d'olive est compris dans une composition nutraceutique.

4. Utilisation selon la revendication 3, **caractérisée en ce que** l'extrait d'olive est consommé environ 20 minutes avant l'exercice.

5. Utilisation d'un extrait d'olive contenant de l'hydroxytyrosol, afin d'augmenter, chez l'homme en bonne santé, les taux de glutathion dans les muscles associés à l'exercice.

6. Utilisation selon la revendication 5, dans laquelle la quantité d'hydroxytyrosol dans l'extrait d'olive se trouve entre 1 mg et 250 mg, et préférablement entre 1 mg et 100 mg.

7. Utilisation selon la revendication 5 ou 6, **caractérisée en ce que** l'extrait d'olive est compris dans une composition nutraceutique.

8. Utilisation selon la revendication 7, **caractérisée en ce que** l'extrait d'olive est consommé environ 20 minutes avant l'exercice.
